# FASCICULE DE BREVET EUROPEEN

(11) **EP 0 482 022 B1**
(45) Date de publication et mention de la délivrance du brevet: **02.06.1993**
(21) Numéro de dépôt: 90909592.9
(22) Date de dépôt: 03.07.1990
(51) Int. Cl.: A61F 6/14

(54) **DISPOSITIF DE FIXATION D'UN DISPOSITIF ANTICONCEPTIONNEL A LA PAROI DE LA MATRICE**
VORRICHTUNG ZUR BEFESTIGUNG EINER EMPFÄNGNISVERHÜTENDEN VORRICHTUNG AN DER GEBÄRMUTTERWAND
DEVICE FOR ATTACHING A CONTRACEPTIVE TO A UTERUS WALL

(30) Priorité: 12.07.1989 BE 8900760
(43) Date de publication de la demande: 29.04.1992
(73) Titulaire: Wildemeersch, Dirk, B-8300 Knokke-Heist (BE)
(72) Inventeur: Wildemeersch, Dirk, B-8300 Knokke-Heist (BE)
(74) Mandataire: Prignot, Jean
(86) Numéro de dépôt international: BE9000038
(87) Numéro de publication internationale: WO9100714

(56) Documents cités:
- BE-A- 899 286
- US-A- 3 598 115
- US-A- 3 888 975
- US-A- 3 954 103

## Description

### Dispositif de fixation d'un dispositif anticonceptionnel à la paroi de la matrice.

La présente invention a pour objet un dispositif de fixation d'un dispositif anticonceptionnel à la paroi de la matrice, du type constitué d'un fil, rendu solidaire du dispositif anticonceptionnel, et d'un organe de retenue implantable dans le tissu de la matrice, solidaire du fil.

L'intérêt des dispositifs anti-conceptionnels fixés à la paroi de la matrice est d'abord apparu dans le domaine du placement de stérilets au cours de la période suivant immédiatement l'accouchement ou période du post-partum. La contraception intrautérine est en effet considérée par les experts comme un moyen contraceptif efficace, peu coûteux et temporaire, ce dernier terme devant être entendu dans le sens où, une fois le dispositif intra-utérin retiré, les grossesses sont à nouveau possibles. Par ailleurs, la période du post-partum est considérée comme une période stratégiquement importante pour l'insertion d'un dispositif intra-utérin, ceci étant particulièrement vrai dans les pays où les femmes ont relativement peu de contacts avec des services médicaux. En effet, lors d'un accouchement, la motivation est grande pour avoir recours à des moyens anticonceptionnels; en outre, à ce moment, l'insertion d'un dispositif anticonceptionnel est aisée, indolore, ne cause pas de saignements supplémentaires, et peut être assurée par l'infirmière ou la sage-femme. Un autre avantage, si l'insertion du dispositif intrautérin est réalisée immédiatement après l'accouchement, est que la parturiente reste encore sous surveillance médicale pendant au moins plusieurs heures après l'insertion. En outre, le placement d'un dispositif intra-utérin n'interfère pas avec l'allaitement, et ne requiert pas non plus l'intervention d'un personnel médical autre que celui intervenant déjà pour l'accouchement.

Toutefois, immédiatement après l'accouchement, les dimensions de la cavité utérine et du col de la matrice sont importantes, ce qui entraîne des risques importants de rejet et/ou de déplacement du dispositif intra-utérin.

C'est une des raisons qui ont conduit à la conception de dispositifs intra-utérins fixés à la paroi de la matrice. De tels dispositifs ont été décrits notamment aux brevets US-A-3.954.103 et BE-A-899.286.

Une autre raison de la fixation du dispositif intra-utérin à la paroi de la matrice est que cette méthode permet l'usage de dispositifs intra-utérins extrêmement déformables qui, sans cette fixation, seraient expulsés de la matrice. En effet, un dispositif intra-utérin très déformable est mieux toléré dans la matrice, et risque moins de causer des dégâts à la paroi de la matrice, qu'un dispositif maintenu dans la matrice par sa forme. De tels dispositifs ont été décrits notamment au brevet EU-A-0100924 et au brevet BE-A-901.652.

Divers organes de retenue, implantables dans la paroi de la matrice, ont été décrits dans ces documents antérieurs.

Par ailleurs, il a déjà été proposé au brevet BE-A-899.286 du demandeur d'utiliser comme organe de retenue implantable, assurant la retenue d'un dispositif intra-utérin conventionnel, un élément constituant pointe, rendu solidaire, de toute manière convenable, du fil retenant le dispositif intra-utérin. Ce même brevet BE-A-899.286, qui vise essentiellement le placement de stérilets dans la matrice au cours de la période du post-partum immédiat, prévoit la possibilité de réaliser l'ensemble du dispositif de retenue (fil et organe de retenue implantable) en une matière biodégradable. La raison en est que les dispositifs intra-utérins préconisés dans ce brevet (en V et en T) sont des dispositifs conventionnels, dont la forme géométrique permet la rétention dans une matrice en-dehors de la période du post-partum immédiat, c'est-à-dire lorsque la cavité de la matrice n'est pas dilatée. Dès lors leur fixation à la paroi de la matrice, en-dehors de la période du post-partum, une fois la matrice revenue à des dimensions normales, n'est plus nécessaire. Dans le mode de réalisation de l'organe de retenue en une matière biodégradable, la fixation du stérilet conventionnel ne subsiste donc qu'aussi longtemps que la matrice n'est pas revenue à une dimension normale.
D'autre part, le brevet US-A-3.888.975 prévoit la réalisation d'un dispositif intra-utérin dont la forme, de manière conventionnelle, assure sa rétention dans une matrice de dimensions normales. Ce dispositif est essentiellement prévu pour délivrer une substance dans la matrice au cours d'une période déterminée. Une fois la diffusion du produit réalisée, le dispositif intra-utérin n'a plus d'utilité. Ce brevet prévoit donc d'utiliser, dans la réalisation du dispositif, une matière biodégradable permettant, après la diffusion du produit, la fragmentation du dispositif et son élimination naturelle de la matrice.

Comme dit ci-dessus, un dispositif anticonceptionnel intra-utérin présente l'avantage d'être un dispositif temporaire, c'est-à-dire susceptible d'être retiré. Si l'on veut que cet avantage soit conservé lors de l'utilisation de dispositifs anticonceptionnels fixés à la paroi de la matrice, il faut donc également que la fixation à la paroi de la matrice puisse être libérée. Si, lors de l'insertion et de la fixation du dispositif intra-utérin à la paroi de la matrice, il est nécessairement fait usage d'un dispositif d'introduction et de fixation du dispositif anticonceptionnel, il semble impossible de devoir faire appel à un dispositif de libération de la fixation, qui ne peut en principe s'effectuer que par traction et arrachement. Dans cette mesure, l'organe de retenue implantable dans le tissu de la matrice décrit au brevet EU-A-0100924 semble difficilement pouvoir être retiré du tissu de la matrice sans y causer des dégât importants. Il en va de même de l'arrachement de la pointe illustrée au brevet US-A-3.954.103 et pourtant préconisé dans ce brevet.

C'est la raison pour laquelle, dans ses brevets antérieurs BE-A-899.286 et BE-A-901.652, le demandeur envisage principalement comme organe de retenue implantable dans le tissu de la matrice, une déformation du fil, de faible volume, telle que notamment un noeud formé dans le fil.

Un tel organe de retenue implantable présente en effet l'avantage, d'abord de réaliser une fixation convenable au tissu de la matrice, au moins en dehors de la période du post-partum, et de permettre en outre une libération de la fixation, par arrachement, sans endommager la paroi de la matrice.

L'expérience a toutefois révélé qu'une déformation de faible volume, et de forme convenable pour éviter d'endommager la paroi de la matrice lors de son arrachement, telle qu'un noeud dans le fil, peut être insuffisante pour assurer un ancrage satisfaisant dans le tissu d'une matrice au cours de la période du post-partum immédiat. Le tissu d'une matrice dilatée, immédiatement après l'accouchement, est en effet beaucoup moins ferme que le tissu d'une matrice à l'état normal.

La présente invention a dès lors pour objet un dispositif de fixation d'un dispositif anticonceptionnel à la paroi de la matrice, permettant une fixation sastisfaisante pendant la période du post-partum immédiat, et pouvant être libéré par arrachement, dans la matrice revenue à l'état normal, sans causer de dégâts au tissu de la matrice.

Ce but est atteint, dans un dispositif de fixation d'un dispositif anti-conceptionnel à la paroi de la matrice, constitué d'un fil en matière non-biodégradable destiné à être attaché au dispositif anticonceptionnel, et d'un organe de retenue implantable dans le tissu de la matrice, solidaire du fil, en prévoyant que l'organe de retenue implantable dans le tissu de la matrice est constitué d'un élément permanent, en matière non-biodégradable, et d'un élément temporaire, en matière biodégradable, conférant temporairement à l'organe de retenue implantable une résistance à l'arrachement supérieure à celle de l'élément permanent seul.

Suivant une autre caractéristique de l'invention, l'élément temporaire, en matière biodégradable, de l'organe de retenue implantable, est percé d'un canal traversé par le fil, et est retenu sur le fil par l'élément permanent, en matière non-biodégradable, de l'organe de retenue implantable.

Suivant une autre caractéristique de l'invention, l'élément permanent, en matière non-biodégradable, est noyé dans l'élément temporaire, en matière biodégradable, de l'organe de retenue implantable.

Suivant encore une autre caractéristique de l'invention, l'organe de retenue implantable dans le tissu de la matrice est de forme générale pointue, pour faciliter sa pénétration dans le tissu de la matrice.

Suivant une autre caractéristique de l'invention, l'élément en matière biodégradable de l'organe de retenue implantable dans le tissu de la matrice constitue l'essentiel du volume de l'organe de retenue implantable, est en forme de pointe et est percé d'un canal débouchant dans la région supérieure de la pointe, traversé par le fil, et en ce qu'une déformation dans le fil, constituant l'élément permanent de l'organe de retenue implantable et réalisée, au-delà du canal, à l'extrémité du fil opposée au dispositif anticonceptionnel, assure également le maintien de l'élément en matière biodégradable sur le fil.

Suivant une autre caractéristique de l'invention, la déformation dans le fil, constituant l'élément permanent de l'organe de retenue implantable, est de volume réduit et de forme convenable pour permettre qu'il soit extrait, sous l'effet d'une traction suffisante, du tissu de la matrice sans endommager ce dernier.

Suivant un mode de réalisation préféré de l'invention, la déformation dans le fil, constituant l'élément permanent de l'organe de retenue implantable, est un noeud dans le fil.

Suivant une caractéristique de l'invention, l'élément temporaire en matière biodégradable de l'organe de retenue implantable comporte, dans sa région opposée à la pointe, des moyens de réception de l'extrémité d'une aiguille, en vue de son implantation dans la paroi de la matrice.

L'invention sera mieux comprise en se reportant à la description, en même temps qu'au dessin annexé, qui représente, uniquement à titre d'exemple, divers modes de réalisation de l'invention, et dans lequel:
- les fig. 1 à 3 représentent, en coupe, divers modes de réalisation du dispositif de fixation d'un dispositif anticonceptionnel intra-utérin suivant l'invention à la paroi de la matrice.
- les fig. 4 et 5 représentent, également en coupe, un dispositif de l'invention en place dans la paroi de la matrice, tel qu'il se présente respectivement immédiatement après son introduction dans la paroi de la matrice, au cours de la période du post-partum immédiat, et quelques mois plus tard, dans la matrice revenue à son état normal.
- la fig 6 enfin est une vue complète de l'ensemble constitué du dispositif intra-utérin, de son dispositif de fixation à la paroi de la matrice, et de son dispositif d'extraction.

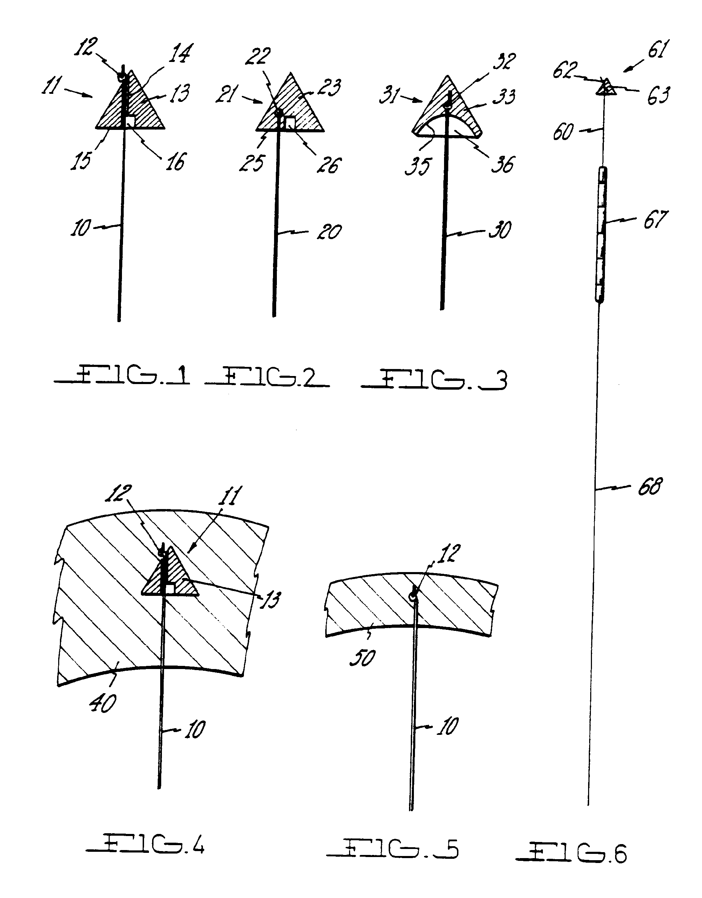

En se reportant au dessin, et plus particulièrement aux modes de réalisation illustrés aux fig. 1 à 3, le dispositif de fixation d'un dispositif anticonceptionnel à la paroi de la matrice se compose suivant l'invention, respectivement d'un fil 10;20;30 en matière non biodégradable, et d'un organe de retenue 11;21;31, implantable dans le tissu de la matrice, solidaire du fil 10;20;30. L'organe de retenue 11;21;31 est constitué d'un élément permanent 12;22;32, en matière non biodégradable, et d'un élément temporaire 13;23;33 en matière biodégradable. En vue de faciliter sa pénétration dans le tissu de la matrice, l'élément temporaire 13;23;33 a la forme générale d'une pointe, et est de dimensions, à sa base, largement supérieures à celles de l'élément permanent 12;22,32, conférant ainsi à l'organe de retenue 11;21;31 une résistance à l'arrachement largement supérieure à celle de l'élément permanent 12;22;32.

Dans le mode de réalisation de la fig. 1, l'élément permanent 12 de l'organe de retenue 11 est un noeud, formé dans le fil 10 après passage de ce dernier au-travers d'un canal 14 percé dans l'élément temporaire 13.

Dans le mode de réalisation de la fig. 2, l'élément permanent 22 de l'organe de retenue 21 est une pièce rapportée, de forme générale sphérique, fixée au fil par soudage/collage, l'ensemble de l'extrémité du fil et de l'élément permanent 22 étant noyé dans l'élément temporaire 23.

Enfin, dans le mode de réalisation de la fig. 3, l'élément permanent 32 de l'organe de retenue 31 est un noeud dans le fil 30, noyé dans l'élément temporaire 33.

En vue de son insertion dans le tissu de la matrice à l'aide d'un dispositif à aiguille de type connu, par exemple du type de ceux décrits et représentés aux brevets BE-A-899.286 ou BE-A-901.562, la base 15;25;35 de l'élément temporaire 13;23;33 comporte des moyens de coopération avec une aiguille. Ces moyens de coopération sont les suivants: dans le mode de réalisation de la fig. 1, un élargissement 16 du canal 14 à partir le la base 15 de l'élément temporaire 13; dans le mode de réalisation de la fig 2, un logement 26 formé dans la base 25, et enfin dans le mode de réalisation de la fig. 3, un creux 36 constitué par la forme concave de la base 35.

L'utilisation de ce dispositif est illustrée en fig. 4 et 5, où l'on a montré un dispositif du type de celui représenté en fig. 1, respectivement en fig. 4 tel qu'il se présente au moment de son implantation dans le tissu de la matrice au cours de la période du post-partum immédiat, et en fig. 5 tel qu'il se présente quelques mois plus tard, dans le tissu de la matrice revenue à sa condition normale.

A la fig. 4, la paroi 40 de la matrice dans la période du post-partum immédiat est épaisse et constituée de tissu de faible résistance. A ce moment, l'organe de retenue implanté 11, constitué de l'élément permanent 12 en matière non biodégradable et de l'élément temporaire 13 en matière biodégradable, a essentiellement le volume et la forme de l'élément temporaire 13 en matière biodégradable, et assure donc un ancrage très résistant dans la paroi 40 de la matrice.

La figure 5 illustre l'ancrage tel qu'il se présente quelques mois après l'implantation représentée en fig. 4. A ce moment, la paroi 50 de la matrice a considérablement diminué d'épaisseur, tandis que le tissu qui la constitue a gagné en résistance mécanique. Dans le même temps, l'élément temporaire 13 de l'organe de retenue implanté 11 s'est dissous et a disparu pour ne laisser subsister que l'élément permanent 12. Ce dernier, vu la consistance du tissu de la matrice, assure un ancrage entièrement satisfaisant du fil 10 dans le tissu de la matrice. Il peut toutefois, moyennant une traction suffisante exercée sur le fil 10, être extrait par arrachement du tissu de la matrice. Vu le volume réduit et la forme générale sensiblement sphérique de l'élément permanent 12, cette extraction par arrachement peut se faire sans causer de dégâts au tissu de la paroi 50 de la matrice.

Enfin, en se reportant à la fig. 6, qui représente un mode de réalisation préféré de l'invention, un dispositif anticonceptionnel intra-utérin comprend, selon l'invention, un dispositif de fixation à la paroi de la matrice constitué d'un fil 60 et d'un organe de retenue 61 implantable dans le tissu de la matrice, cet élément de retenue 61 étant lui-même constitué d'un élément permanent 62 en matière non biodégradable, et d'un élément temporaire 63 en matière biodégradable. Suivant ce mode de réalisation, l'élément permanent 62 est constitué d'un noeud dans l'extémité du fil 60 au-delà de l'élément temporaire 63, tandis qu'en-deçà de l'élément temporaire 63, le fil 60 se poursuit pour recevoir un dispositif anticonceptionnel 67 proprement dit, et que ce même fil 60 se poursuit en-deçà du dispositif anticonceptionnel 67 pour constituer dispositif d'extraction 68.

## Revendications

1. Dispositif de fixation d'un dispositif anticonceptionnel à la paroi de la matrice, constitué d'un fil (10;20;30;60) en matière non-biodégradable, destiné à être attaché au dispositif anticonceptionnel, et d'un organe de retenue (11;21;31;61) implantable dans le tissu de la matrice, solidaire du fil, caractérisé en ce que l'organe de retenue (11;21;31;61) implantable dans le tissu de la matrice, est constitué d'un élément permanent (12;22;32;62), en matière non-biodégradable, et d'un élément temporaire (13;23;33;63), en matière biodégradable, conférant temporairement à l'organe de retenue implantable (11;21;31;61) une résistance à l'arrachement supérieure à celle de l'élément permanent (12;22;32;62) seul.

2. Dispositif suivant 1, caractérisé en ce que l'élément temporaire (13;23;33;63), en matière biodégradable, de l'organe de retenue implantable (11;21;31;61;) est retenu sur le fil (10;20;30;60) par l'élément permanent (12;22;32;62), en matière non-biodégradable, de l'organe de retenue implantable.

3. Dispositif suivant 1, caractérisé en ce que l'élément permanent (22;32), en matière non-biodégradable, est noyé dans l'élément temporaire (23;33), en matière biodégradable, de l'organe de retenue implantable (21;31).

4. Dispositif suivant l'une quelconque des revendications 1 à 3, caractérisé en ce que l'organe de retenue (11;21;31;61) implantable dans le tissu de la matrice, est de forme générale pointue, pour faciliter sa pénétration dans le tissu de la matrice.

5. Dispositif suivant l'une quelconque des revendications 1,2 et 4, caractérisé en ce que l'élément en matière biodégradable (13) de l'organe de retenue (11) implantable dans le tissu de la matrice constitue l'essentiel du volume de l'organe de retenue implantable (11), est en forme de pointe, et est percé d'un canal (14) débouchant dans la région supérieure de la pointe, traversé par le fil (10), et en ce qu'une déformation (12) dans le fil, constituant l'élément permanent de l'organe de retenue implantable et réalisée, au-delà du canal, à l'extrémité du fil opposée au dispositif anticonceptionnel, assure également le maintien de l'élément en matière biodégradable (13) sur le fil.

6. Dispositif suivant l'une quelconque des revendications 1 à 5, caractérisé en ce que la déformation dans le fil (10;20;30), constituant l'élément permanent (12;22;32) de l'organe de retenue implantable, est de volume réduit et de forme généralement sphérique pour permettre qu'il soit extrait, sous l'effet d'une traction suffisante, du tissu de la matrice sans endommager ce dernier.

7. Dispositif suivant 6, caractérisé en ce que la déformation (12;32;62) dans le fil (10;30;60), constituant l'élément permanent de l'organe de retenue implantable (11;31;61), est un noeud dans le fil (10;30;60).

8. Dispositif suivant l'une quelconque des revendications 1 à 7, caractérisé en ce que l'élément temporaire (13;23;33) en matière biodégradable de l'organe de retenue implantable (11;21;31) comporte, dans sa région (15;25;35) opposée à la pointe, des moyens de réception (16;26;36) de l'extrémité d'une aiguille, en vue de son implantation dans la paroi de la matrice.

## Patentansprüche

1. Vorrichtung zur Befestigung einer Empfängnisverhütungsvorrichtung an der Gebärmutterwand, bestehend aus einem Faden (10;20;30;60) aus biologisch nicht abbaubarem Material zur Befestigung an der Empfängnisverhütungsvorrichtung und einem in das Gebärmuttergewebe implantierbaren, fest mit dem Faden verbundenen Rückhalteorgan (11;21;31;61), dadurch gekennzeichnet, daß
das in das Gebärmuttergewebe implantierbare Rückhalteorgan (11;21;31;61) aus einem permanenten Element (12;22;32;62) aus biologisch nicht abbaubarem Material und einem temporären Element (13;23;33;63) aus biologisch abbaubarem Material besteht, das dem implantierbaren Rückhalteorgan (11;21;31;61) vorübergehend eine Ausreißfestigkeit verleiht, die größer ist als diejenige des permanenten Elementes (12;22;32;62) allein.

2. Vorrichtung nach Anspruch 1, dadurch gekennzeichnet, daß das temporäre Element (13;23;33;63) aus biologisch abbaubarem Material des implantierbaren Rückhalteorgans (11;21;31;61) durch das permanente Element (12;22;32;62) aus biologisch nicht abbaubarem Material des implantierbaren Rückhalteorgans auf dem Faden (10;20;30;60) festgehalten wird.

3. Vorrichtung nach Anspruch 1, dadurch gekennzeichnet, daß das permanente Element (22;32) aus biologisch nicht abbaubarem Material in dem permanenten Element (23;33) aus biologisch abbaubarem Material des implantierbaren Rückhalteorgans (21;31) verknotet ist.

4. Vorrichtung nach einem der Ansprüche 1 bis 3, dadurch gekennzeichnet, daß das in das Gebärmuttergewebe implantierbare Rückhalteorgan (11;21;31;61) von allgemein spitzer Form ist, um sein Eindringen in das Gebärmuttergewebe zu erleichtern.

5. Vorrichtung nach einem der Ansprüche 1, 2 und 4, dadurch gekennzeichnet, daß das aus biologisch abbaubarem Material bestehende Element (13) des in das Gebärmuttergewebe implantierbaren Rückhalteorgans (11) den Hauptumfang des implantierbaren Rückhalteorgans (11) ausmacht, die Form einer Spitze aufweist und von einem Kanal (14) durchdrungen ist, der in die obere Region der Spitze mündet, durch den der Faden (10) verläuft und daß eine Verformung (12) des Fadens, die das permanente Element des implantierbaren Rückhalteorgans darstellt und die außerhalb des Kanals am gegenüberliegenden Ende des Fadens der Empfängnisverhütungsvorrichtung vorgesehen ist, auch die Halterung des Elementes aus biologisch abbaubarem Material (13) auf dem Faden gewährleistet.

6. Vorrichtung nach einem der Ansprüche 1 bis 5, dadurch gekennzeichnet, daß die das permanente Element (12;22;32) des implantierbaren Rückhalteorgans darstellende Verformung in dem Faden (10;20;30) von reduziertem Umfang und im allgemeinen kugelförmig ist, damit er mit genügendem Zug aus dem Gebärmuttergewebe herausgezogen werden kann, ohne dieses zu verletzen.

7. Vorrichtung nach Anspruch 6, dadurch gekennzeichnet, daß die das permanente Element des implantierbaren Rückhalteorgans (11;31;61) darstellende Verformung (12;32;62) in dem Faden (10;30;60) ein Knoten in dem Faden (10;30;60) ist.

8. Vorrichtung nach einem der Ansprüche 1 bis 7, dadurch gekennzeichnet, daß das temporäre Element (13;23;33) aus biologisch abbaubarem Material des implantierbaren Rückhalteorgans (11;21;31) zur Implantation in die Gebärmutterwand in der der Spitze gegenüberliegenden Region (15;25;35) Aufnahmevorrichtungen (16;26;36) für die äußerste Spitze einer Nadel aufweist.

## Claims

1. A device for fixing a contraceptive device to the wall of the uterus, consisting of a thread (10;20;30;60) of non-biodegradable material, designed to be attached to the contraceptive device, and a retaining member (11;21;31;61) implantable in the tissue of the uterus and integral with the thread, characterized in that the retaining member (11;21;31;61) implantable in the tissue of the uterus consists of a permanent element (12;22;32;62), of non-biodegradable material, and a temporary element (13;23;33;63), of biodegradable material, temporarily conferring to the implantable retaining member (11;21;31;61) greater resistance to pulling out than that of the permanent element (12;22;32;62) alone.

2. A device according to claim 1, characterized in that the temporary element (13;23;33;63), of biodegradable material, of the implantable retaining member (11;21;31;61) is held on the thread (10;20;30;60) by the permanent element (12;22;32;62), of non-biodegradable material, of the implantable retaining member.

3. A device according to claim 1, characterized in that the permanent element (22;32), of non-biodegradable material, is buried in the temporary element (23;33), of biodegradable material, of the implantable retaining member (21;31).

4. A device according to any one of claims 1 to 3, characterized in that the retaining member (11;21;31;61) implantable in the tissue of the uterus, is generally pointed in form to facilitate its penetration into the tissue of the uterus.

5. A device according to any one of claims 1, 2 and 4, characterized in that the element of biodegradable material (13) of the retaining member (11) implantable in the tissue of the uterus constitutes the major part of the size of the implantable retaining member (11), is pointed in shape and is pierced by a channel (14), opening in the upper part of the point, through which the thread (10) passes, and in that a deformation (12) in the thread, constituting the permanent element of the implantable retaining member and formed beyond the channel at the opposite end of the thread from the contraceptive device, also ensures that the element of biodegradable material (13) is held on the thread.

6. A device according to any one of claims 1 to 5, characterized in that the deformation in the thread (10;20;30), constituting the permanent element (12;22;32) of the implantable retaining member, is smaller in volume and of generally spherical form to allow its withdrawal, under the effect of sufficient pulling, from the tissue of the uterus without damaging the latter.

7. A device according to claim 6, characterized in that the deformation (12;32;62) in the thread (10;30;60), constituting the permanent element of the implantable retaining member (11;31;61), is a knot in the thread (10;30;60);

8. A device according to any one of claims 1 to 7, characterized in that the temporary element (13;23;33) of biodegradable material of the implantable retaining member (11;21;31) comprises, in the area (15;25;35) thereof remote from the point, means (16,26,36) for receiving the end of a needle, with a view to its implatation in the wall of the uterus.
